# EUROPEAN PATENT APPLICATION

(11) **EP 4 741 494 A1**
(43) Date of publication of application: **13.05.2026**
(21) Application number: 24840042.6
(22) Date of filing: 08.07.2024
(51) Int. Cl.: C12N 5/071, A61K 35/42, A61P 29/00, A61P 11/00

(54) **METHOD FOR CULTURING LUNG-DERIVED STEM CELLS, AND USE THEREOF**

(30) Priority: 07.07.2023 KR 20230088609
(71) Applicant: Hierabio Inc., Seongnam-si, Gyeonggi-do 13605 (KR)
(72) Inventor: JEON, Jeong Hwa, Seoul 03938 (KR); SHIN, Ji Young, Seongnam-si, Gyeonggi-do 13595 (KR); KIM, Hee Jung, Seoul 04778 (KR); LEE, Seung Jin, Seoul 07333 (KR)
(74) Representative: Weickmann & Weickmann PartmbB
(86) International application number: PCT/KR2024/009702
(87) International publication number: WO 2025/014230

(57) **Abstract**

Provided are a method of culturing lung-derived stem cells and use thereof, and to a method of culturing lung-derived stem cells; lung-derived stem cells prepared by the method; a pharmaceutical composition for preventing or treating hyperinflammatory diseases, the pharmaceutical composition comprising, as an active ingredient, the prepared lung-derived stem cells, a culture thereof, or cells differentiated from the lung-derived stem cells; and a quasi-drug composition.

According to the culture method of the present disclosure, stem cells may be effectively isolated and obtained from lung tissues, and the obtained lung-derived stem cells have excellent pluripotency and self-renewal capacity, and excellent effects of alleviating, improving, and treating hyperinflammatory diseases, and therefore, they may be usefully applied for various purposes.

## Description

### [Technical Field]

The present disclosure relates to a method of culturing lung-derived stem cells and use thereof, and to a method of culturing lung-derived stem cells; lung-derived stem cells prepared by the method; a pharmaceutical composition for preventing or treating hyperinflammatory diseases, the pharmaceutical composition comprising, as an active ingredient, the prepared lung-derived stem cells, a culture thereof, or cells differentiated from the lung-derived stem cells; and a quasi-drug composition.

### [Background Art]

Stem cells refer to cells in a pre-differentiation stage before they differentiate into individual cells that make up a tissue, and to cells that can proliferate infinitely in an undifferentiated state and have the potential to differentiate into various types of tissue cells through specific differentiation stimulation.

According to their differentiation potency, stem cells are largely divided into embryonic stem cells (ES cells) and adult stem cells (tissue-specific stem cells). Embryonic stem cells are stem cells isolated from the inner cell mass (ICM), which will develop into a fetus, of a blastocyst embryo, the initial stage in which the fertilized egg is formed but is not yet implanted in the endometrium, and are cells that have the potential to differentiate into cells of all tissues.

In contrast, tissue-specific stem cells are stem cells specific to each organ that appear in the stage in which the embryonic development process progresses and each organ of the embryo is formed, and their differentiation potency is generally limited (multipotent) only to the cells that make up the tissue. Representative tissue-specific stem cells comprise hematopoietic stem cells, which exist in the bone marrow, and mesenchymal stem cells, which differentiate into connective tissue cells other than blood cells. Hematopoietic stem cells differentiate into various blood cells such as erythrocytes, leukocytes, etc., and mesenchymal stem cells differentiate into osteoblasts, chondroblasts, adipocytes, and myoblasts, etc.

Recently, since the success of the isolation of embryonic stem cells from humans, interest in their clinical application has grown. The application of stem cells that is receiving the most attention is the use of stem cells as a cell source for cell replacement therapy.

The technology of isolating/culturing lung-derived stem cells of the present disclosure is a technology utilizing a hydrogel-based niche-preserving, self-renewal-induced isolation technology for stem cell isolation, and is a novel technology that enables the *ex vivo* survival/culture of lung tissue and the direct isolation of highly pure cells from the tissue without enzymatic treatment.

Existing methods of isolating lung-derived stem cells rely on enzymatic isolation methods using collagenase, etc. (Korean Patent Publication No. 10-2014-0075469 A), and these methods inevitably damage cell surface proteins, affecting cell performance. However, the present isolation method is distinct from the existing methods, and has an advantage of being superior in terms of *ex vivo* expansion and cell performance.

### [Disclosure]

### [Technical Problem]

Cells isolated using existing methods are able to be expanded *ex vivo* only for 2 passages to 3 passages (up to 5 passages). However, cells isolated using a method of the present disclosure exhibit superior performance, such as no cell senescence even after passages of 8 or more. Therefore, long-term passage culture is possible for mass production.

Under this background, the present inventors have conducted extensive research to develop novel stem cells. As a result, they found that lung-derived stem cells prepared by the culture method of the present disclosure exhibit superior *ex vivo* expansion, cell performance, and differentiation potency, and accordingly, they may be used for the treatment of hyperinflammatory diseases, thereby completing the present disclosure.

### [Technical Solution]

An object of the present disclosure is to provide a method of culturing lung-derived stem cells using a lung tissue and a hydrogel.

Another object of the present disclosure is to provide lung-derived stem cells prepared by the above method.

Still another object of the present disclosure is to provide a pharmaceutical composition for preventing or treating hyperinflammatory diseases, the pharmaceutical composition comprising, as an active ingredient, the lung-derived stem cells, a culture thereof, or cells differentiated from the lung-derived stem cells.

Still another object of the present disclosure is to provide a quasi-drug composition for preventing or improving hyperinflammatory diseases, the quasi-drug composition comprising, as an active ingredient, the lung-derived stem cells, a culture thereof, or cells differentiated from the lung-derived stem cells.

Still another object of the present disclosure is to provide a composition for culturing lung-derived stem cells, the composition comprising a lung tissue and a hydrogel.

Still another object of the present disclosure is to provide a kit for culturing lung-derived stem cells, the kit comprising the composition for culturing stem cells.

### [Advantageous Effects]

According to a culture method of the present disclosure, stem cells may be effectively isolated and obtained from lung tissues, and the obtained lung-derived stem cells have excellent pluripotency and self-renewal capacity, and excellent effects of alleviating, improving, and treating hyperinflammatory diseases; therefore, they may be usefully applied for various purposes.

### [Brief Description of the Drawing]

FIG. 1A shows the results of migration and proliferation patterns of lung tissue cells embedded in a wound-mimicking hydrogel after performing centrifugation-based lung tissue selection pretreatment;
FIG. 1B shows the results of cell migration and proliferation patterns without performing centrifugation-based lung tissue selection pretreatment;
FIG. 2A shows the morphology of lung-derived stem cells and their population doubling time at passages 5 to 7;
FIG. 2B shows the morphology of lung-derived stem cells isolated without lung tissue selection pretreatment and their population doubling time at passage 6;
FIG. 3 shows the results of immunophenotypic analysis of lung-derived stem cells;
FIG. 4 shows the results of examining colony-forming capacity of lung-derived stem cells (HB-LSC: HB-Lung derived stem cell);
FIG. 5A shows the results of examining the differentiation potency of lung-derived stem cells into adipocytes;
FIG. 5B shows the results of examining the differentiation potency of skin-derived stem cells into osteocytes;
FIG. 6 shows the results of evaluating the biodistribution of lung-derived stem cells in an animal model (LPS: Lipopolysaccharide; HB-LSC: HB-Lung derived stem cell);
FIG. 7 shows the results of examining the anti-inflammatory activity of lung-derived stem cells (LPS: Lipopolysaccharide; HB-LSC: HB-Lung derived stem cell);
FIG. 8 shows the results of examining the anti-inflammatory activity of lung-derived stem cells (BLM: Bleomycin; HB-LSC: HB-Lung derived stem cell);
FIG. 9 shows the results of examining the anti-fibrotic activity of lung-derived stem cells (BLM: Bleomycin; HB-LSC: HB-Lung derived stem cell); and
FIG. 10 shows the results of evaluating the inflammation-related cytokine secretion ability of lung-derived stem cells.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

To achieve the above objects, one aspect of the present disclosure provides a method of culturing lung-derived stem cells using a lung tissue and a hydrogel, and lung-derived stem cells cultured by the method.

As used herein, the term "lung-derived stem cells" may comprise all stem cells derived from the lung tissue, and specifically, may be lung-derived mesenchymal stem cells, but is not limited thereto.

Specifically, the method of culturing lung tissue-derived stem cells comprises the steps of (a) embedding the lung tissue in the hydrogel and culturing the tissue to obtain a culture; and (b) decomposing the hydrogel in the obtained culture to recover stem cells that have migrated from the lung tissue into the hydrogel and proliferated therein.

In the present disclosure, the hydrogel refers to a three-dimensional network structure formed by crosslinking hydrophilic polymers via covalent or noncovalent bonds. As the lung tissue is three-dimensionally embedded in the hydrogel, the hydrogel may provide physical support for the lung tissue, and at the same time, may provide an extracellular matrix function that allows lung-derived stem cells present in the lung tissue to migrate into and proliferate in the hydrogel.

In addition, the hydrogel of the present disclosure is preferably a phase transitional hydrogel that may be converted from a solution state to a sol and a gel for hydrogel-supported three-dimensional culture, and specifically, it may be any one or more selected from the group consisting of collagen, gelatin, chondroitin, hyaluronic acid, alginic acid, Matrigel^{™}, chitosan, peptide, fibrin, polyglycolic acid (PGA), polylactic acid (PLA) and polyethylene glycol (PEG), polyacrylamide, but is not limited thereto.

In the step (a), the culture of the hydrogel in which lung tissue is embedded may be performed after immersing the hydrogel in which lung tissue is embedded, in a common medium known in the art to be suitable for stem cell culture.

In the step (b), only the hydrogel may be selectively decomposed in order to recover lung-derived stem cells that have migrated into/proliferated in the hydrogel after the hydrogel-supported three-dimensional culture step of the step (a).

The hydrogel may be decomposed through one or more enzymes selected from the group consisting of collagenase, gelatinase, urokinase, streptokinase, tissue plasminogen activator (TPA), plasmin, and hyaluronidase. In addition, the step (a) may comprise a pretreatment step of selecting lung tissue fragments. Specifically, the step (a) may be performed after the pretreatment step of selecting lung tissue fragments, and the pretreatment step may be selecting a supernatant through centrifugation after fragmenting the lung tissue.

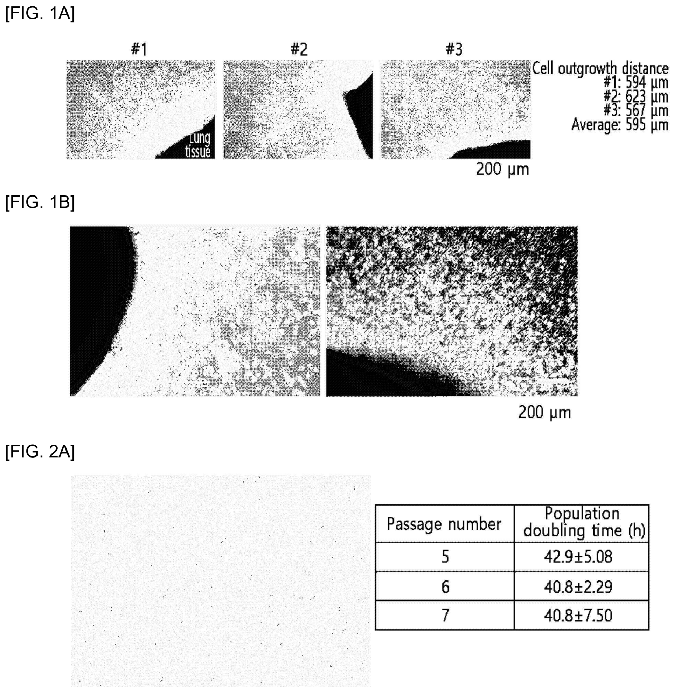

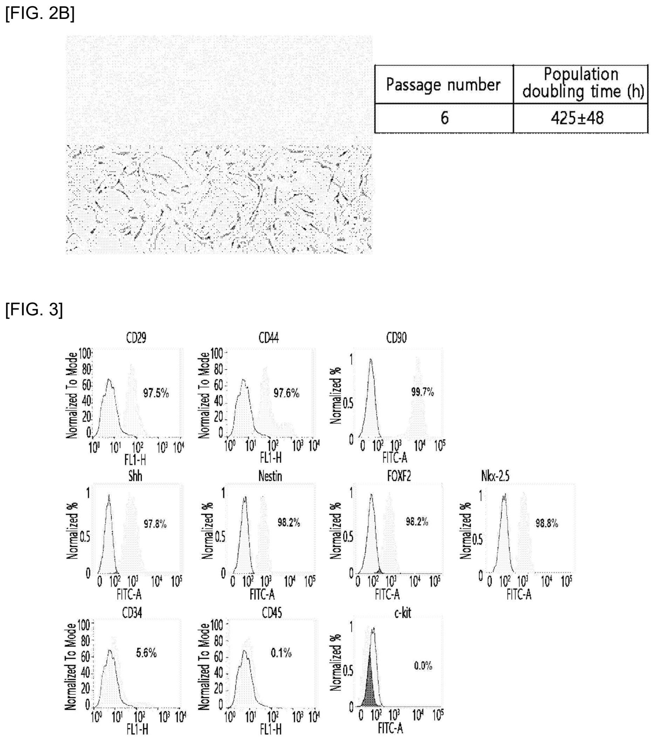

In one embodiment of the present disclosure, when the centrifugation-based tissue selection, which is the pretreatment step of selecting the lung tissue, was not performed, the cell morphology was not spindle-shaped but mixed, and at the passage number of 6, the PDT was 425 hours, implying that the proliferation ability of the cells was almost lost, and thus it was confirmed that the cell morphology was not uniform and the cell proliferation ability was reduced, as compared to the case where the pretreatment step was performed (FIGS. 1 and 2).

Meanwhile, the lung-derived stem cells which are prepared by the method of preparing lung-derived stem cells provided in the present disclosure may exhibit immunological characteristics of expressing CD90, CD29, or CD44 on the cell surface, but not expressing hematopoietic cell phenotypes CD34 and CD45, but are not limited thereto.

Further, the lung-derived stem cells may exhibit immunological characteristics of expressing Sonic hedgehog protein (Shh), Forkhead Box F2 (FOXF2), NK2 Homeobox 5 (Nkx-2.5), or neuroepithelial stem cell protein (Nestin) on the cell surface, but not expressing Mast/stem cell growth factor receptor kit (c-kit).

Still another aspect of the present disclosure provides a pharmaceutical composition for preventing or improving hyperinflammatory diseases, the pharmaceutical composition comprising, as an active ingredient, the lung-derived stem cells, a culture thereof, or cells differentiated from the lung-derived stem cells.

As used herein, the term "hyperinflammatory diseases" refers to any disease or condition caused by hyperinflammatory responses, particularly inflammatory conditions characterized by an overproduction of cytokines (cytokine storm). Such diseases include, but are not limited to, respiratory inflammation, systemic inflammatory response due to respiratory inflammation, pneumonia, acute respiratory distress syndrome (ARDS), pulmonary fibrosis, cystic fibrosis, pulmonary alveolar proteinosis, lung cancer, pulmonary embolism, pulmonary edema, cytokine storm, and inflammation caused by respiratory viruses.

Specifically, the inflammation caused by respiratory viruses may be caused by SARS coronavirus-2 (SARS-CoV-2) or SARS virus infection, but is not limited to.

The lung-derived stem cells of the present disclosure may exhibit prophylactic or therapeutic effects on hyperinflammatory diseases by alleviating or improving hyperinflammatory diseases.

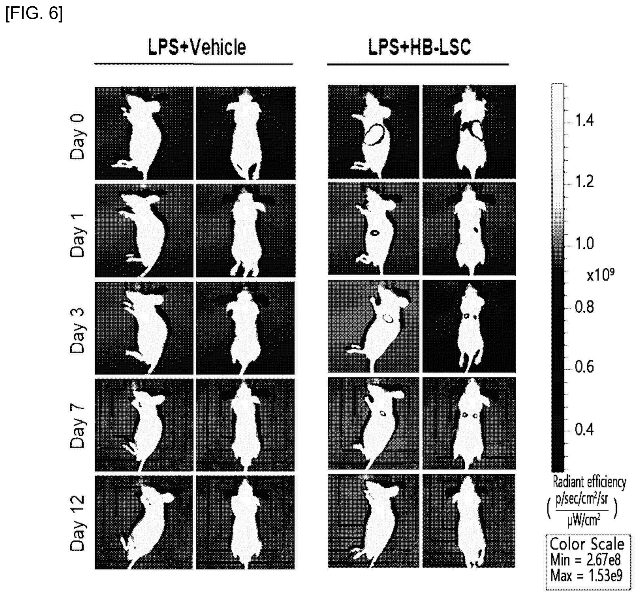

In one embodiment of the present disclosure, it was confirmed that the lung-derived stem cells injected into a mouse disease model of pulmonary fibrosis and a mouse disease model of pulmonary inflammation were specifically distributed in the lungs, and their anti-fibrotic and anti-inflammatory effects were confirmed, respectively (FIGS. 6 to 10).

As used herein, the term "culture" or "culture solution" may refer to a culture obtained during or after culture of lung-derived stem cells in a medium, or a supernatant thereof, a concentrate thereof, or a lyophilized product thereof.

As used herein, the term "pharmaceutical composition" refers to a product prepared for the purpose of preventing or treating a disease, and may be administered in various oral and parenteral dosage forms upon actual clinical administration, and in the case of being formulated, the pharmaceutical composition may be prepared using diluents or excipients commonly used, such as fillers, extenders, binders, wetting agents, disintegrants, surfactants, etc.

In addition, depending on each formulation, pharmaceutically acceptable additives may be further comprised, and in this regard, as the pharmaceutically acceptable additives, starch, gelatinized starch, microcrystalline cellulose, lactose, povidone, colloidal silicon dioxide, calcium hydrogen phosphate, lactose, mannitol, taffy, gum arabic, pregelatinized starch, corn starch, powdered cellulose, hydroxypropyl cellulose, Opadry, sodium starch glycolate, carnauba wax, synthetic aluminum silicate, stearic acid, magnesium stearate, aluminum stearate, calcium stearate, white sugar, dextrose, sorbitol, and talc, etc. may be used. The pharmaceutically acceptable additives according to the present disclosure may be comprised in an amount of 0.1 part by weight to 90 parts by weight with respect to the composition.

In addition to the lung-derived mesenchymal stem cells, the pharmaceutical composition may further comprise one or more common pharmaceutically acceptable inert carriers, for example, preservatives, analgesics, solubilizers, or stabilizers, etc. in the case of injections, and bases, excipients, lubricants, or preservatives, etc. in the case of preparations for topical administration.

The pharmaceutical composition of the present disclosure may be administered as an individual therapeutic agent or in combination with other therapeutic agents, and may be administered sequentially or simultaneously with traditional therapeutic agents, and may be administered a single time or multiple times. It is important to administer the pharmaceutical composition in a minimum amount that may afford the maximum effect without causing side effects, taking all the factors into consideration, and this minimum amount may be easily determined by a person skilled in the art.

In addition, the pharmaceutical composition provided in the present disclosure may further comprise various ingredients that assist prevention or treatment of hyperinflammatory diseases, maintain the activity of the lung-derived stem cells, or promote differentiation of the lung-derived stem cells, in addition to the lung-derived stem cells, and for example, may further comprise anti-inflammatory agents, stem cell mobilization factors, growth inducing factors, etc.

As used herein, the term "improvement" may refer to any action by which the progression of hyperinflammatory diseases is delayed or symptoms thereof are alleviated by administering the composition according to the present disclosure to an individual.

As used herein, the term "prevention" may refer to any action by which the occurrence of hyperinflammatory diseases is restrained or retarded by administering the composition according to the present disclosure to an individual.

As used herein, the term "treatment" may refer to any action by which symptoms of hyperinflammatory diseases have taken a turn for the better or been modified favorably by administering the composition of the present disclosure to an individual suspected of having hyperinflammatory diseases.

Still another aspect of the present disclosure provides a method of treating hyperinflammatory diseases, the method comprising the step of administering to an individual the composition comprising the lung-derived stem cells, a culture thereof, or cells differentiated from the skin-derived stem cells as an active ingredient.

The terms "lung-derived stem cells", "culture thereof", "cells differentiated from the lung-derived stem cells", "hyperinflammatory diseases", and "treatment" are as described above.

As used herein, the term "administering" means introducing the composition into an individual in an appropriate manner. Specifically, the composition comprising the lung-derived stem cells of the present disclosure, the culture thereof, or cells differentiated from the lung-derived stem cells as an active ingredient, or the pharmaceutical composition comprising the same may be applied to a local site or administered as an injectable formulation, but is not limited thereto.

Further, the composition may be used in combination with other pharmaceutical compositions which may be used for hyperinflammatory diseases.

As used herein, the term "individual" means any animal such as rats, mice, livestock, etc., comprising humans, which has or may develop hyperinflammatory diseases. The animal may be a human as well as a mammal, such as a cow, horse, sheep, pig, goat, camel, antelope, dog, cat, etc., in need of prevention or treatment of symptoms similar thereto, but is not limited thereto.

The composition of the present disclosure may be administered in a pharmaceutically effective amount.

The term "pharmaceutically effective amount" means an amount sufficient to treat a disease with a reasonable benefit/risk ratio applicable to medical treatment, and the effective dosage level may be determined depending on factors comprising the type and severity of the disease, the individual's age and sex, the drug's activity, sensitivity to the drug, time of administration, route of administration and rate of excretion, period of treatment, drugs used concurrently, and other factors well known in the medical field.

The composition may be administered as an individual therapeutic agent or in combination with other therapeutic agents, and may be administered sequentially or simultaneously with traditional therapeutic agents. The composition may be administered a single time or multiple times. It is important to administer the composition in a minimum amount that may afford the maximum effect without causing side effects, taking all the factors into consideration, and this minimum amount may be easily determined by a person skilled in the art.

Further, the composition may be administered orally or parenterally (e.g., intravenously, subcutaneously, intraperitoneally, or topically) depending on the desired method, and the dosage varies depending on the patient's condition and weight, degree of disease, drug form, and route and time of administration, but may be appropriately selected by a person skilled in the art. As a specific example, the composition may generally be administered once or several times a day, but the preferred dosage may be appropriately selected by a person skilled in the art depending on the individual's condition and weight, degree of disease, drug form, and route and period of administration.

Still another aspect of the present disclosure provides a quasi-drug composition for preventing or improving hyperinflammatory diseases, the quasi-drug composition comprising, as an active ingredient, the lung-derived stem cells, a culture thereof, or cells differentiated from the lung-derived stem cells.

As used herein, the term "quasi-drug composition" is an article used for the purpose of treating, alleviating, healing, or preventing diseases in humans or animals, which corresponds to one of textiles, rubber products or similar articles, articles that weakly act on the human body or do not directly act on the human body and are not instruments or machines and similar articles, and preparations used for sterilization, deinsectization, and similar purposes to prevent infection, and refers to articles used for the purpose of diagnosing, healing, alleviating, treating or preventing diseases in humans or animals which are neither instruments, machines, nor devices, and articles used for the purpose of having a pharmacological effect on the structure and function of humans or animals excluding those that are not instruments, machines, or devices, and may be specifically a preparation for external use or a personal care product.

Still another aspect of the present disclosure provides a composition for culturing lung-derived stem cells, the composition comprising a lung tissue and a hydrogel, and a kit for culturing lung-derived stem cells, the kit comprising the composition.

In addition, the kit for culturing lung-derived stem cells may comprise the composition for culturing stem cells and various components such as solutions and devices necessary for the culture of the stem cells.

Still another aspect of the present disclosure provides use of the composition comprising the lung-derived stem cells, a culture thereof, or cells differentiated from the lung-derived stem cells as an active ingredient in preventing, treating, or improving hyperinflammatory diseases.

Definitions of the terms are as described above.

### [Mode for Carrying Out the Invention]

Hereinafter, the present disclosure will be described in more detail with reference to exemplary embodiments. However, these exemplary embodiments are only for illustrating the present disclosure, and the scope of the present disclosure is not intended to be limited by these exemplary embodiments.

### Example 1. Three-Dimensional Culture of Lung Tissue in Wound-Mimicking Hydrogel

Lung tissues extracted from an animal or donated from a human were washed three times with PBS (Gibco) and once with DMEM (low glucose, Gibco). The washed lung tissues were cut into fragments with a size of 0.5 mm to 5 mm. Among the tissue fragments, those with hematoma areas were excluded, followed by centrifugation at 1500 rpm for 1 minute under refrigerated conditions, and the tissue fragments in the supernatant were collected and used for subsequent tissue culture. Thereafter, 5 mL of fibrinogen solution (0.25% w/v fibrinogen (Green Cross), 200 µg/mL tranexamic acid (Shin Poong Pharmaceutical) in DMEM) was added. After an equal amount of thrombin solution (0.5 IU/mL thrombin (Green Cross) in DMEM) was added, the mixture was immediately transferred to a 100 mm culture vessel to ensure that the lung tissue fragments embedded in the hydrogel were evenly distributed in the culture vessel. When necessary, forceps were used to secure tissue fragment spacing. Sufficient gelation was achieved by performing incubation at room temperature for 5 minutes and at 37°C for 1 hour. A growth medium (90% low DMEM:F12=1:1, 10% fetal bovine serum, 20 ng/mL epidermal growth factor, 5 ng/mL basic fibroblast growth factor, 10 ng/mL insulin-like growth factor, and 10 mg/mL gentamicin) supplemented with 100 µg/mL tranexamic acid was added, and the culture medium was replaced every 1 hour for 3 hours. Thereafter, the tissue was cultured while changing the growth medium once every 2 days to 3 days.

### Example 2. Recovery and Amplification Culture of Lung-Derived Stem Cells Migrated into and Grown in Hydrogel

Lung tissue was cultured for 5 days to 10 days by the method described in Example 1. After the hydrogel was washed with warm PBS three times for 3 minutes, 30% FBS/DMEM was added, followed by stirring on an orbital shaker (30 rpm). 1 hour later, the supernatant was discarded, and then 30% FBS/low DMEM supplemented with 1 µg/mL alteplase (Boehringer Ingelheim) was added, and the gel was selectively decomposed to obtain cells. The obtained cells were cultured by a common culture method, and the growth medium was replaced every 2 days to 3 days, and subculture was performed when the cell density reached 80%. During initial culture, cells were seeded at a density of 4,000 cells/cm² and passaged every 6 days. Through the number of obtained cells, the population doubling time according to the number of passages was measured. Furthermore, the number of cells unstained by trypan blue staining was counted as the cell number. The population doubling time (PDT) was calculated using the following equation and is shown in FIG. 3: PDT = [days / (logN₂ - logN₁)] / log2, where N₁ = 4,000 cells and N₂ = number of cells after 6 days.

The tissue culture results and the cell isolation and culture results obtained according to the above method are illustrated in FIGS. 1 and 2.

As shown in FIG. 1, cell migration and proliferation from the tissue were observed from day 2 of culture, and an increase in the cell number due to cell migration and proliferation was observed. The outgrowth distance of cells from the tissue on day 4 of culture was 595 µm on average, and isolation and culture were performed at that point. When centrifugation-based tissue selection was not performed, spindle-shaped and spherical cells were mixed, as shown in the results on day 5 of culture in FIG. 1B.

Furthermore, when isolated lung-derived stem cells were cultured as shown in FIG. 2A, they adhered in a spindle shape, and when cultured to 7 passages, a population doubling time (PDT) was 40.8 hours, confirming that the proliferation potential was maintained at a predetermined level. As shown in FIG. 2B, when tissue selection was not performed, the cell morphology was mixed, not spindle-shaped, and at passage number 6, PDT was 425 hours, confirming that the cell proliferation potential was almost lost.

In other words, the above results confirmed that the cell morphology was uneven and the cell proliferation potential was reduced, as compared to the case where the pretreatment step of selecting lung tissue fragments was performed.

### Example 3. Examination of Immunophenotype of Isolated and Cultured Lung-Derived Stem Cells

In order to examine the immunophenotype of lung-derived stem cells (hereinafter, HB-lung-derived stem cells: HB-LSCs) isolated and cultured by the method of Example 2, cells cultured in a T75 flask were recovered and suspended in 3% bovine serum albumin (Sigma)/PBS. The cell suspensions were reacted at room temperature for 30 minutes with the following fluorescent-labeled antibodies, respectively. FITC, PerCP, Alexa Fluor 488 or Alexa Fluor 594-labeled antibodies: CD29 (Bio-rad, 1:10), CD44 (Invitrogen, 1:100), CD90 (abcam, 1:100), CD34 (Santa Cruz Biotechnology, 1:5), CD45 (Novus Biologicals, 1:20), Nestin (Novus Biologicals, 1:100), Nkx-2.5 (Santa Cruz Biotechnology, 1:40), c-Kit (Invitrogen, 1:20), FOXF2 (LSBio, 1:100), Shh (Bioss, 1:100). After the reaction, centrifugation was performed, followed by washing and measurement using a flow cytometer (BD) (FIG. 3).

As a result, as shown in FIG. 3 and Table 1, it was found that mesenchymal stem cell markers, CD29, CD44, and CD90 were all positively expressed at 90% or more on the surface of isolated and cultured lung-derived stem cells. Hematopoietic cell markers CD34 and CD45 were confirmed to be negatively expressed. It was confirmed that lung tissue-specific markers, Shh and FOXF2 were positively expressed, c-kit was negatively expressed, and other specific markers, Nkx-2.5 and Nestin, were positively expressed.

**[Table 1]**

| **Categories** | **Markers** | **Other Name** | **Cell Populati on (%)** | **Phenoty pe*** |
|---|---|---|---|---|
| MSC markers | CD29 | Integrin beta-1 | 97.5% | Positive |
| | CD44 | Hyaluronic acid receptor | 97.6% | Positive |
| | CD90 | Thy-1 membrane glycoprotein | 99.7% | Positive |
| Lung | Shh | Sonic hedgehog protein | 97.8% | Positive |
| specific markers | FOXF2 | Forkhead Box F2 | 98.2% | Positive |
| | c-kit | Mast/stem cell growth factor receptor kit | 0.0% | Negative |
| Other specific markers | Nkx-2.5 | NK2 Homeobox 5 | 98.8% | Positive |
| | Nestin | neuroepithelial stem cell protein | 98.2% | Positive |
| Hematopoi etic markers | CD34 | Hematopoietic progenitor cell antigen | 5.6% | Negative |
| | CD45 | Receptor-type tyrosine-protein phosphatase C | 0.1% | Negative |

| | | | | |
|---|---|---|---|---|
| * Positive: ≥ 90% cell population, Negative: ≤ 10% cell population | | | | |

### Example 4. Examination of Colony-Forming Capacity of Isolated and Cultured HB-Lung-Derived Stem Cells

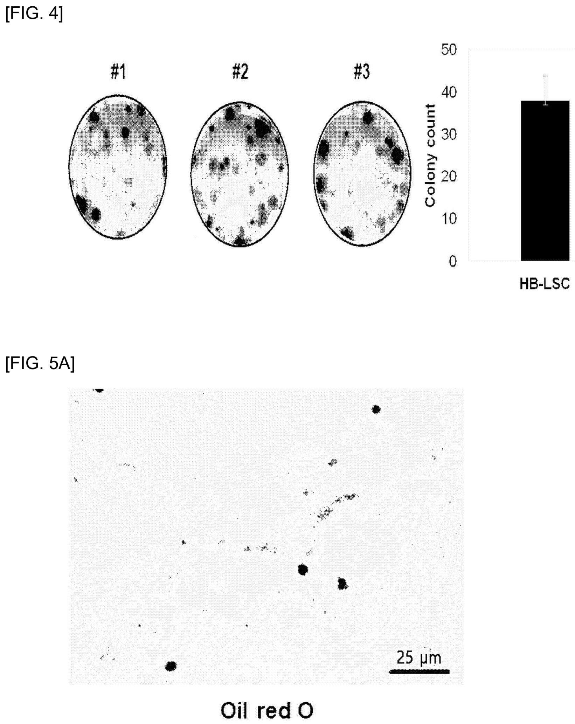

In order to examine the colony-forming capacity of the HB-lung-derived stem cells, lung-derived stem cells (7 passages) were seeded in a 6-well plate at 1,000 cells/well. The cells were cultured for 14 days while replacing the growth medium every 3 days. The cells were washed with PBS and fixed with 4% paraformaldehyde/PBS (Sigma) at room temperature for 15 minutes. The fixed cells were stained with 0.5% crystal violet/20% methanol at room temperature for 15 minutes. After all remaining solutions were removed, the cells were washed with distilled water, the stained colonies were observed, and the results are shown in FIG. 4. It was found that when the cells were seeded at 1,000 cells/well, 35.7 colonies on average were formed. The colony-forming capacity of HB-lung-derived stem cells was above a certain level, indicating that the stem cells possessed self-renewal ability.

### Example 5. Examination of Differentiation Potency of HB-Lung-Derived Stem Cells

In order to evaluate the differentiation potency of the obtained HB-lung-derived stem cells into adipocytes, cells were seeded at a density of 1 × 10⁴ cells/cm² and cultured in a growth medium until reaching 80% confluency, then the medium was changed using a StemPro^{™} Adipogenesis differentiation kit (Gibco, cat. A1007001), and culture was performed for 14 days to 21 days while replacing the medium every 3 days. The cells were fixed with 4% PFA for 15 minutes and reacted with 60% isopropanol for 5 minutes, then the solutions were all removed. The cells were stained with 60% Oil Red O (Sigma)/ethanol at room temperature for 15 minutes to examine fat accumulation in the cytoplasm (FIG. 5A).

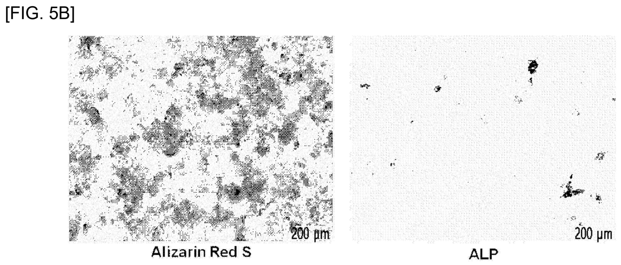

Further, in order to evaluate the differentiation potency of the HB-lung-derived stem cells into osteocytes, cells were seeded at a density of 5 × 10³ cells/cm² and cultured in a growth medium until reaching 80% confluency, then the medium was changed using a StemPro^{™} Osteogenesis differentiation kit (Gibco, cat. A1007201), and culture was performed while replacing the medium every 3 days. The cells were fixed with 4% PFA, washed and stained with Alizarin red solution (pH 4.2) for 10 minutes to 15 minutes, and then Alizarin red S staining of the cells was examined. Additionally, cells were fixed with 4% PFA for 60 seconds, treated with BCIP/NBT substrate solution (Sigma), and then stained for 10 minutes under light-blocking conditions to examine the activity of alkaline phosphatase (ALP) (FIG. 5B).

In other words, the results confirmed that HB-lung-derived stem cells obtained by the culture method of the present disclosure possessed pluripotency.

### Example 6. Preparation of Pulmonary Fibrosis Mouse Disease Model and Efficacy Evaluation

Bleomycin (3 mg/g) dissolved in physiological saline was instilled (50 µL/animal) into the trachea of BALB/c nude mice (6wks, Male) using a mouse intratracheal catheter. To ensure consistent induction of pulmonary fibrosis, the mice were shaken up and down three times before recovery from anesthesia. After bleomycin administration, body weight and general symptoms were observed once every 2 days to 3 days. One week later, surviving animals were administered cells (5 x 10⁵ cells/200 µL in DMEM (Phenol Red Free)) via the tail vein using an insulin syringe (30G). After cell administration, body weight and general symptoms were observed once every 2 days to 3 days. 14 days after cell administration, autopsy was performed, and the animals were visually examined for any adverse reactions. At autopsy, visual observation confirmed that no abnormal reactions were observed in organs other than the lungs, which were the site of disease induction.

### Example 7. Preparation of Pulmonary Inflammation Mouse Disease Model and Distribution/Efficacy Evaluation

LPS (5 mg/g) dissolved in physiological saline was instilled (50 µL/animal) into the trachea of BALB/c nude mice (6wks, Male) using a mouse intratracheal catheter (Kent Scientific Inc.). To ensure consistent induction of pulmonary inflammation, the mice were shaken up and down three times before recovery from anesthesia. 1 day after LPS administration, surviving animals were administered cells (5 x 10⁵ cells/200 µL in DMEM (Phenol Red Free)) via the tail vein using an insulin syringe (30G). After cell administration, body weight and general symptoms were observed once every 2 days to 3 days. 13 days after cell administration, autopsy was performed, and the animals were visually examined for any adverse reactions. To perform image-based biodistribution evaluation, DiR-stained cells were administered, and immediately after administration, on Day 1, Day 3, Day 7, and Day 12, IVIS (IVIS Lumina Series III, Perkin Elmer) images were taken. It was confirmed that DiR-stained cells administered through the tail vein were distributed in the lungs and liver immediately after administration. It was confirmed that DiR-stained cells were mainly distributed in the lungs from Day 1 to one week after administration and disappeared by Day 12 after administration. Based on the above results, it was confirmed that when administered through the tail vein for the treatment of lung disease, cells may be specifically distributed in the lungs (FIG. 6).

### Example 8. Examination of Anti-Fibrotic and Anti-Inflammatory Activity of HB-Lung-Derived Stem Cells

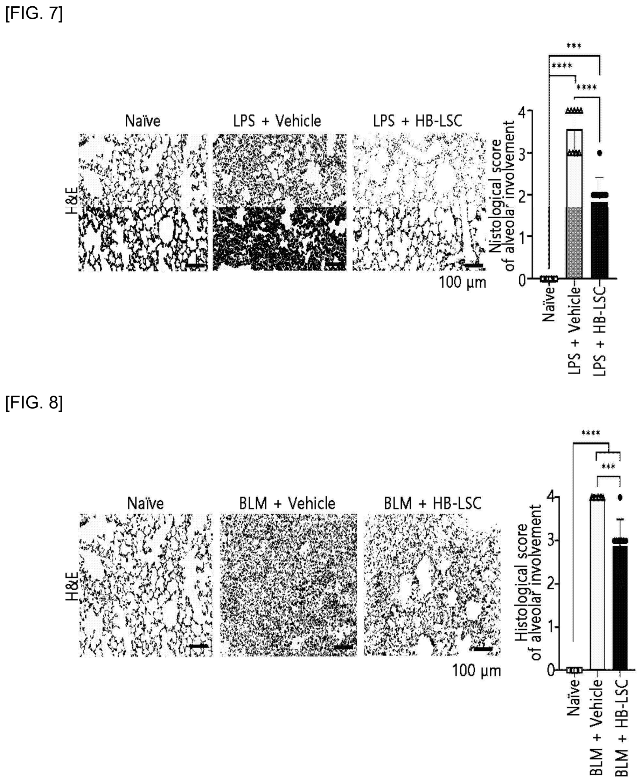

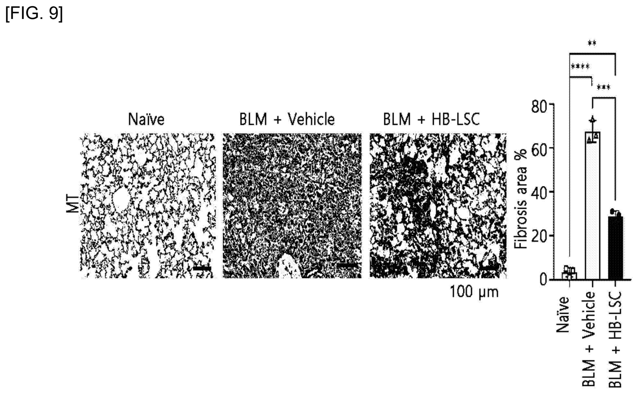

The lung tissues of the mouse disease models of Examples 6 and 7 were excised and fixed in 10% formalin for 24 hours, and after fixation, tissue processing and paraffin blocks were prepared. To examine the histopathological changes of the lungs, the blocks were sectioned to 4 µm and stained with Hematoxylin & Eosin (H&E) to evaluate the histological changes and the degree of inflammatory cell infiltration in the lungs, as shown in the table below. Based on the table, the histopathological changes in the lungs of each group were scored. As a result, it was confirmed that inflammation was significantly improved in the group in which cells were administered to the LPS-induced inflammation disease model, as compared to the vehicle group (FIG. 7). It was also confirmed that the inflammatory cell infiltration was significantly improved in the group in which cells were administered to the bleomycin-induced lung fibrosis disease model, as compared to the vehicle group (FIG. 8). Anti-fibrotic activity was evaluated through Masson's Trichrome (MT) staining, and the fibrotic area was quantified using ImageJ Software (US National Institute of Health Program, USA). Although there was a significant difference in the cell-administered group, as compared to the normal group, the fibrotic area was significantly reduced in the cell-administered group, as compared to the vehicle group, and it was confirmed that the fibrotic area in the lung was reduced by 30% or more (p<0.001) (FIG. 9). In other words, the above results confirmed that HB-lung-derived stem cells possess anti-inflammatory and anti-fibrotic activity.

**[Table 2]**

| Score | Alveolar Involvement |
|---|---|
| 0 | None |
| 1 | Mild (patchy increased cellularity without septal thickening) |
| 2 | Moderate (increased cellularity with septal thickening) |
| 3 | Severe (25-50% visualized lung with increased cellularity/thickening) |
| 4 | Diffuse (>50% visualized lung with increased cellularity/ thickening) |

### Example 9. Evaluation of Cytokine Secretion Ability of HB-Lung-Derived Stem Cells

HB-lung-derived stem cells were cultured to 80% confluency, washed once with PBS, and cultured for 3 days after replacing with low-serum medium (2% FBS/DMEM). Culture media on Days 1 and 3 were collected and centrifuged at 1,500 rpm for 5 minutes to obtain supernatants, which were analyzed by Proteome Profiler Array (Mouse Cytokine Array Panel A, R&D Systems).

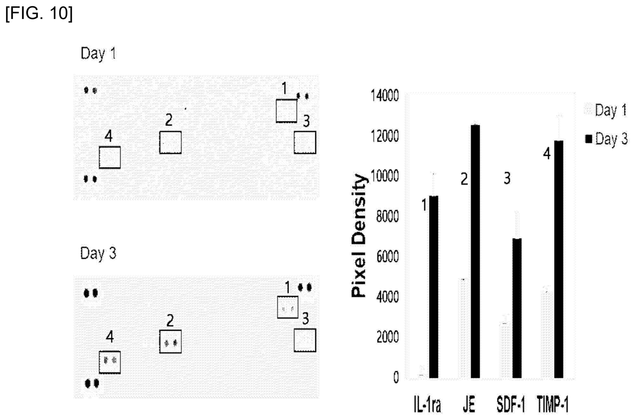

As shown in FIG. 10, it was confirmed that HB-lung-derived stem cells secreted IL-1ra, JE, SDF-1, and TIMP-1. In particular, JE, SDF-1, and TIMP-1 were all detected in the culture media on Days 1 and 3, and the secretion amount of IL-1ra on Day 3 increased 53.1-fold, as compared to that on Day 1. Furthermore, this unique secretory profile is characterized by the non-secretion of major inflammatory cytokines, including interleukin-6 (IL-6), IL-12, and IL-18, tumor necrosis factor alpha (TNF-α), interferon gamma (IFNγ) and granulocyte-macrophage colony stimulating factor (GM-CSF), IFN-gamma, IL-12, *etc.*

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of culturing lung-derived stem cells, the method comprising the steps of:
(a) embedding a lung tissue in a hydrogel and culturing the tissue to obtain a culture; and
(b) decomposing the hydrogel in the obtained culture to recover stem cells that have migrated from the lung tissue into the hydrogel and proliferated therein.

2. The method of claim 1, wherein the step (a) comprises a pretreatment step of selecting lung tissue fragments.

3. The method of claim 2, wherein the pretreatment step is selecting a supernatant through centrifugation after fragmenting the lung tissue.

4. A lung-derived stem cell prepared by the method of claim 1.

5. The lung-derived stem cell of claim 4, wherein the lung-derived stem cells exhibit immunological characteristics of expressing CD90, CD29, or CD44 on the cell surface, but not expressing hematopoietic cell phenotypes CD34 and CD45.

6. The lung-derived stem cell of claim 4, wherein the lung-derived stem cells exhibit immunological characteristics of expressing Sonic hedgehog protein (Shh), Forkhead Box F2 (FOXF2), NK2 Homeobox 5 (Nkx-2.5), or neuroepithelial stem cell protein (Nestin) on the cell surface, but not expressing Mast/stem cell growth factor receptor kit (c-kit).

7. A pharmaceutical composition for preventing or treating hyperinflammatory diseases, the pharmaceutical composition comprising, as an active ingredient, the lung-derived stem cells prepared by the method of claim 1, a culture thereof, or cells differentiated from the lung-derived stem cells.

8. The pharmaceutical composition of claim 7, wherein the hyperinflammatory diseases are any one or more selected from respiratory inflammation, systemic inflammatory response due to respiratory inflammation, pneumonia, acute respiratory distress syndrome (ARDS), pulmonary fibrosis, cystic fibrosis, pulmonary alveolar proteinosis, lung cancer, pulmonary embolism, pulmonary edema, cytokine storm, and inflammation caused by respiratory viruses.

9. The pharmaceutical composition of claim 7, wherein the hyperinflammatory diseases are pneumonia or pulmonary fibrosis.

10. The pharmaceutical composition of claim 7, wherein the lung-derived stem cells exhibit immunological characteristics of expressing CD90, CD29, or CD44 on the cell surface, but not expressing hematopoietic cell phenotypes CD34 and CD45.

11. The pharmaceutical composition of claim 7, wherein the lung-derived stem cells exhibit immunological characteristics of expressing Sonic hedgehog protein (Shh), Forkhead Box F2 (FOXF2), NK2 Homeobox 5 (Nkx-2.5), or neuroepithelial stem cell protein (Nestin) on the cell surface, but not expressing Mast/stem cell growth factor receptor kit (c-kit).

12. A quasi-drug composition for preventing or improving hyperinflammatory diseases, the quasi-drug composition comprising, as an active ingredient, the lung-derived stem cells prepared by the method of claim 1, a culture thereof, or cells differentiated from the lung-derived stem cells.

13. A composition for culturing the lung-derived stem cells of claim 1, the composition comprising a lung tissue and a hydrogel.

14. A kit for culturing lung-derived stem cells, the kit comprising the composition for culturing stem cells of claim 13.

15. Use of a composition comprising lung-derived stem cells prepared by the method of claim 1, a culture thereof, or cells differentiated from the lung-derived stem cells as an active ingredient in preventing, treating, or improving hyperinflammatory diseases.
